# EUROPEAN PATENT APPLICATION

(11) **EP 2 068 151 A1**
(43) Date of publication of application: **10.06.2009**
(21) Application number: 07023607.0
(22) Date of filing: 05.12.2007
(51) Int. Cl.: G01N 33/68

(54) **Soluble amyloid precursor proteins in cerebrospinal fluid as biomarkers of Alzheimer's disease**

(71) Applicant: Friedrich-Alexander-Universität Erlangen-Nürnberg, 91054 Erlangen (DE)
(72) Inventor: Wiltfang, Jens, Prof. Dr. med., 91056 Erlangen (DE); Lewczuk, Piotr, Priv.-Doz. Dr. med., 91341 Röttenbach (DE); Kornhuber, Johannes, Prof. Dr. med., 90491 Nürnberg (DE)
(74) Representative: Rehberg Hüppe + Partner

(57) **Abstract**

An *ex vivo* method of diagnosing Alzheimer's disease comprising the steps of analysing a cerebrospinal fluid (CSF) sample taken from a patient with regard to a concentration of at least one amyloid precursor protein (APP) product selected from the group consisting of soluble forms of amyloid precursor protein α and β (sAPPα and sAPPβ); comparing the concentration of the at least one APP product with a threshold-value for the concentration of the respective one APP product; and indicating a probability that the patient has or is prone to Alzheimer's disease, if the concentration of the at least one APP product exceeds the threshold-value for the concentration of the respective one APP product.

## Description

### FIELD OF THE INVENTION

The invention generally relates to an *ex vivo* method of diagnosing Alzheimer's disease comprising the steps of the preamble of independent claim 1. Within this aspect, the invention relates to a method of using an assay for determining a concentration of at least one amyloid precursor protein (APP) product in a cerebrospinal fluid (CSF) sample. Further, the invention relates to a kit for carrying out the *ex vivo* method comprising the features of the preamble of claim 8.

The patient from which the CSF sample is taken will be a mammal. Preferably, the mammal is a human.

### PRIOR ART

With the increase of the percentage of older population in the Western industrialized countries, the incidence of dementia disorders increases simultaneously, resulting in an increasing burden on the health care system. In case of Alzheimer's disease (AD), the increasing number of patients has not so far resulted in the achievement of accurate standards of *durante vitam* diagnosis. Although sensitivity of clinical diagnosis is relatively high (93%), specificity may be lower, being reported as 55% in a multicenter clinical-autopsy study (Mayeux, 1998). In expert hands the clinical diagnosis of AD is predictive of AD pathology in 80%-90% of cases. Nevertheless, very early diagnosis of AD, and differential diagnosis of unusual presentations of patients with dementia remains difficult on clinical grounds.

With the introduction of potentially successful treatments for dementias that were previously considered to be irreversible (for a review, see: (Klafki et al., 2006)), the need for an early and differential diagnosis of dementia becomes even more urgent (The Working Group on: "Molecular and Biochemical Markers of Alzheimer's Disease", 1998; Wiltfang et al., 2005). Since cerebrospinal fluid (CSF) is in direct contact with the central nervous system, it is obvious that any changes in biochemical composition of brain parenchyma should be predominantly reflected in CSF. Lumbar puncture is an easy procedure, with a low incidence of complications. In a large study by Andreasen et al (Andreasen et al., 2001), only 4.1% of all patients experienced post-lumbar headache, and an even smaller incidence of 2% was reported in a study by Blennow et al (Blennow et al., 1993). It is therefore reasonable to postulate that lumbar puncture is a feasible, moderately invasive procedure, and CSF analysis could possibly improve current clinical and neuroimaging-based approaches to diagnosis. Nevertheless, the low and limited amount of the material obtained with a lumbar puncture requires very precise planning of the laboratory tests to be performed. The growing number of possibly important biomarkers to be analyzed in a limited volume of the CSF makes techniques of a simultaneous analysis of several parameters in a single small-volume CSF sample (multiplexing) a method of choice in the future (Lewczuk et al., 2007; Olsson et al., 2005).

Multiparametric CSF-based neurochemical dementia diagnostics (CSF-NDD) is used to support the diagnosis of Alzheimer's disease (AD). In CSF-NDD it is, for example, known to measure the concentration of phospho-Tau in a CSF sample taken from a patient, to compare this concentration with a threshold for phospho-Tau, and to regard a concentration in excess of the threshold as an indication that there is an increased probability that the patient has or is prone to have AD. Thus, an *ex vivo* method comprising the steps of the preamble of claim 1 is known.

Meso Scale Discovery (Gaithersburg, USA) has been offering multiplexing assays for determining CSF concentrations of sAPPα and sAPPβ since 2005 (Tynan et al., 2005). sAPPα and sAPPβ are amyloid precursor protein (APP) products resulting from a cleavage with α-secretase or β-secretase, respectively. APP cleavage with β-secretase and γ-secretase results in the release of multiple peptides including Aβ40 and Aβ42. Aβ42 is a major component of amyloid plaques, the characteristic protein deposits found in AD patients. The Meso Scale Discovery assay is a multiplex assay to analyze APP products. For example, sAPPα and sAPPβ levels can be simultaneously quantified in a single CSF sample, brain homogenate or cell culture supernatant in 3-4 hours.

There are different low throughput methods to measure sAPP (based on electrophoresis) that did not show differences in sAPP CSF concentrations between AD and non-AD patients.

### PROBLEM OF THE INVENTION

It is the problem of the present invention to provide a new *ex vivo* method enhancing CSF-based neurochemical dementia diagnostics (CSF-NDD) with regard to Alzheimer's disease, and a kit for carrying out this new method.

### SOLUTION

The problem of the present invention is solved by an ex vivo method of diagnosing Alzheimer's disease comprising the features of claim 1, by a method of using an immunoassay using an electrochemiluminescent reporter for determining a concentration of at least one amyloid precursor protein (APP) product in a cerebrospinal fluid (CSF) sample comprising the features of claim 7, and a kit for carrying out the first method comprising the features of claim 8.

Preferred embodiments of the present invention are defined in the dependent claims.

### SUMMARY OF THE INVENTION

In an *ex vivo* method of diagnosing Alzheimer's disease according to the present invention at least one amyloid precursor protein (APP) product analyzed in a cerebrospinal fluid (CSF) sample taken from a patient as a biomarker of Alzheimer's disease (AD) is selected from the group consisting of soluble forms of amyloid precursor protein α and β (sAPPα and sAPPβ).

Despite the facts that assays for determining CSF concentrations of sAPPα and sAPPβ have been available since 2005 (Tynan et al., 2005) and that there is an urgent need for additional parameters to be used in CSF-based neurochemical dementia diagnostics (CSF-NDD), no correlations between the occurrence of AD and altered CSF levels of sAPPα and sAPPβ have been reported up to now. Particularly, Tynan et al (Tynan et al., 2005) mention sAPPα or sAPPβ in the context of AD, but do not even indicate any possible correlation of sAPPα or sAPPβ levels in CSF with AD.

The results of a study presented below nevertheless indicate that sAPPα and sAPPβ are both well suited biomarkers of AD in CSF. Further, this study revealed the very important fact that the CSF concentrations sAPPα and sAPPβ do not vary to a relevant extent as a result of different storage conditions. Particularly no relevant variation was observed due to different non-cryogenic storage conditions. Thus, the samples may be easily sent to a laboratory carrying out the new method without the need of express transport or special cooled transport containers. Further, the method of the invention may be executed at any time after the respective CSF sample was taken. This includes executing the method of the invention even with regard to old existing CSF samples taken from patient to retrospectively monitor the progress of AD, for example. It is also of relevance that the study presented below indicates a strong positive correlation of the sAPPα and sAPPβ CSF concentrations both in AD and in non AD patients. This allows for an internal counter-check of the diagnostic results obtained with the new method, if both the sAPPα and sAPPβ CSF concentrations are determined, which is therefore strongly preferred. The strong positive correlation of the sAPPα and sAPPβ CSF concentrations both in AD and in non AD patients, however, do not allow for a simple interpretation why these concentrations are increased with AD. Said the other way round: it is most surprising that both sAPPα and sAPPβ are increased in CSF of AD patients to the same percental extent.

The strong positive correlation of the sAPPα and sAPPβ CSF concentrations also allows for only determining one of these two concentrations or the total concentration of both sAPPα and sAPPβ in the new *ex vivo* method without loosing essential information for AD diagnostics..

The threshold value for the concentration of sAPPα applied in the new method may be easily obtained by comparing the sAPPα concentrations in CSF samples taken from AD patients with those in CSF samples taken from non-AD controls. Typically, the threshold value for sAPPα will be between 110 ng/mL and 125 ng/mL. Preferably, it is between 115 ng/mL and 120 ng/mL and more preferably at about 117.4 mg/mL.

The threshold value for the concentration of sAPPβ applied in the new method may be easily obtained by comparing the sAPPβ concentrations in CSF samples taken from AD patients with those in CSF samples taken from non-AD controls. Typically, the threshold value for sAPPβ will be between 175 ng/mL and 190 ng/mL, preferably between 180 ng/mL and 185 ng/mL and more preferably at about 181.8 mg/mL.

The application of carefully selected threshold values as cutoff values in the new method results in both a high sensitivity and specificity in diagnosing AD. The threshold for the concentration of sAPPα will easily display a sensitivity of at least 60 %. Preferably its sensitivity is at least 65 %, and more preferably it is at least 68 %. The same threshold will easily display a specificity of at least 75 %. Preferably its specificity is at least 80 %, and more preferably it is at least 85 %.

Despite the strong positive correlation of both sAPP concentrations in CSF, the threshold for the concentration of sAPPβ may even achieve a higher sensitivity in the new method. Easily it displays a sensitivity of at least 65 %. Preferably its sensitivity is at least 70 %, and more preferably it is at least 75 %. The same threshold will easily display a specificity of at least 75 %. Preferably its specificity is at least 80 %, and more preferably it is at least 85 %.

In executing the method a commercially available immunoassay using an electrochemiluminescent reporter for determining a concentration of at least one amyloid precursor protein (APP) product selected from the group consisting of soluble forms of amyloid precursor protein α and β (sAPPα and sAPPβ) in a cerebrospinal fluid (CSF) sample may be used. This immunoassay may, for example, be the multiplexing immunoassay available fro Meso Scale Discovery. Such an immunoassay allows for a 'high-throughput method' of measuring the two biomarkers sAPPα and sAPPβ of the present invention. For example, up to 40 samples can be measured (in duplicate) within about 4 hours using such an immunoassay.

A kit according to the present invention for carrying out the new method comprising an assay for determining a concentration of at least one amyloid precursor protein (APP) product selected from the group consisting of soluble forms of amyloid precursor protein α and β (sAPPα and sAPPβ) in a cerebrospinal fluid (CSF) sample, and an indicator indicating whether the concentration of the at least one APP product exceeds a threshold-value for the concentration of the respective one APP product. Preferably the kit comprises an assay for the CSF concentrations of both sAPPα and sAPPβ, and an indicator indicating whether the CSF concentrations of both sAPPα and sAPPβ exceed their respective threshold-value.

Other features and advantages of the present invention will become apparent to one with skill in the art upon examination of the following drawings and the detailed description. It is intended that all such additional features and advantages be included herein within the scope of the present invention, as defined by the claims.

### SHORT DESCRIPTION OF THE DRAWINGS

The invention can be better understood with reference to the following drawings.
- **Fig. 1**: illustrates the stability of the sAPPα (panel a) and sAPPβ (panel b) concentrations in CSF samples stored under different conditions. The results are normalized for the results from the aliquot stored at -80 °C unthawed. * p<0.01; + p<0.05; NS, not significant.
- **Fig. 2**: illustrates the correlation between sAPPα and sAPPβ in the CSF samples.
- **Fig. 3**: displays CSF concentrations of sAPPα (panel a) and sAPPβ (panel b) in groups of patients with NDD positive and NDD negative results. * p<0.001.
- **Fig. 4**: displays CSF concentrations of sAPPα (panel a) and sAPPβ (panel b) in groups of patients with clinical diagnoses supported by corresponding NDD results. * p<0.05; NS, not significant.

### DETAILED DESCRIPTION

### 1. Overview

The results of a multicenter study to test analytic and diagnostic performance of soluble forms of amyloid precursor protein α and β (sAPPα and sAPPβ) in the cerebrospinal fluid (CSF) of patients with different forms of dementing conditions are presented. CSF samples were collected from 188 patients with early dementia (MMSE ≥20 in majority of cases) and mild cognitive impairment (MCI) in twelve gerontopsychiatric centers, and the clinical diagnoses were supported by neurochemical dementia diagnostics (NDD) tools: CSF amyloid β peptides, tau, and phospho-tau. sAPPα and sAPPβ were measured with multiplexing method based on electrochemiluminescence. sAPPα and sAPPβ CSF concentrations correlated with each other with very high correlation ratio (R=0.96, p<0.001). Highly significantly increased sAPPα and sAPPβ CSF concentrations in patients with NDD characteristic for Alzheimer's disease (AD) compared to those with NDD negative results were observed. sAPPα and sAPPβ highly significantly separated patients with AD, whose diagnosis was supported by NDD findings (sAPPα: cut off, 117.4ng/mL, sensitivity, 68%, specificity, 85%, p<0.001; sAPPβ: cut off, 181.8ng/mL, sensitivity, 75%, specificity, 85%, p<0.001), from the patients clinically assessed as having other dementias and supported by NDD untypical for AD. sAPPα and sAPPβ are thus to be regarded as biomarkers supporting clinical diagnosis of Alzheimer's disease.

### 2. Materials and Methods

### 2.1. Patients and lumbar punctures

The study was approved by the ethics committees of all the participating universities, and all patients and/or their relatives gave their informed consent.

Twelve German gerontopsychiatric university departments participated in the project (hftp://www.kompetenznetz-demenzen.de), recruiting a total of n=188 patients with early dementias (in a great majority of cases MMSE ≥20) or mild cognitive impairment (MCI). Patients of this study were categorized according to a) clinical and neuropsychological, and b) neurochemical criteria:

Patients with early AD (D-AD; n=69) were diagnosed according to the criteria of ICD-10, and the National Institute of Neurological and Communicative Disorders and the Stroke-Alzheimer's Disease and Related Disorders Association (NINCDS-ADRDA) (McKhann et al., 1984). Patients with other early dementias (D-O; n=27) fulfilled the criteria of corresponding disorders (vascular dementia, n=3; frontotemporal degeneration, n=10; dementia with Lewy bodies, n=4; Parkinson's disease, n=1; corticobasal degeneration, n=1; progressive supranuclear palsy, n=1; other and unclassified, n=7). Cognitive dysfunction was assessed with CERAD neuropsychological test battery (Thalmann et al., 1998), WMS-R Logical Memory (Härting et al., 2000), Trail Making Test (Reitan, 1979), and Clock Drawing Test (Shulman et al., 1986). Functional decline was assessed using informant questionnaires B-ADL (Hindmarch et al., 1998), and IQCODE (Jorm, 1994). Distinction of MCI and dementia was based on CDR rating (Morris, 1993). A diagnosis of MCI corresponded to a CDR score of 0.5, a diagnosis of mild dementia corresponded to a CDR score of 1.0. Severity of dementia was graded according to the Mini Mental State Examination (Folstein et al., 1975), with the score was available for 185 patients.

Patients with MCI fulfilled the modified criteria of Petersen et al (Petersen et al., 2001), and were subdivided into two groups: those with clinical, neuropsychological, and neuroradiological signs suggesting development of AD (MCI-AD; n=54), and those with the signs suggesting development of other dementias (MCI-O; n=38).

In the second categorization, patients were divided according to the neurochemical findings in the CSF: the group with pathologic results of neurochemical dementia diagnosis (NDD+; n=103) characterizing with CSF decreased amyloid β x-42/x-40 concentration ratio (Aβ Ratio; cut off, 0.11 (Lewczuk et al., 2004c)) accompanied by increased CSF concentration of phospho-Tau181 (pTau181; cut off 70 pg/mL), and the group with normal results of neurochemical dementia diagnosis (NDD-; n=48) characterizing with normal amyloid β ratio (i.e. higher that 0.11) accompanied by normal pTau181 (below 50pg/mL). Note that n=37 patients with 'borderline' CSF pTau181 concentration (50-70pg/mL) were not considered for this categorization.

Patients of the study were further categorized according combined clinical and neurochemical data, i.e. D-AD, D-O, MCI-AD, and MCI-O patients were selected fulfilling corresponding NDD criteria: positive or negative values of Aβ Ratio and pTau181: D-AD/NDD+, n=54; D-O/NDD-, n=7; MCI-AD/NDD+, n=30; and MCI-O/NDD-, n=20. The demographic data of the patients are shown in the Table 1.

**Table 1: Demographic data of the patients**

| Group (n) | Age (± SD) | M+F | ε4/total genotyped | MMSE (± SD) |
|---|---|---|---|---|
| D-AD (69) | 70.64 ± 8.0 | 30 + 39 | 34/55 | 22.9 ± 2.6 |
| D-O (27) | 68.63 ± 8.0 | 19+8 | 10/22 | 23.0 ± 3.6 |
| MCI-AD (54) | 68.26 ± 8.2 | 37 + 17 | 23 / 44 | 26.5 ± 2.2 |
| MCI-O (38) | 65.08 ± 7.9 | 21 + 17 | 10/35 | 27.2 ± 2.4 |
| NDD positive (103) | 69.26 ± 8.1 | 48 + 55 | 55 / 85 | 24.11 ± 3.0 |
| NDD negative (48) | 65.83 ± 8.1 | 35+13 | 10/40 | 26.17 ± 3.2 |
| D-AD/NDD+ (54) | 69.8 ± 7.5 | 22 + 32 | 31 / 44 | 22.9 ± 2.5 |
| D-O/NDD- (7) | 64.9 ± 10.5 | 6+1 | 1/6 | 23.3 ± 3.0 |
| MCI-AD/NDD+ (30) | 69.1 ± 9.2 | 18 + 12 | 16/26 | 26.0 ± 2.2 |
| MCI-O/NDD- (20) | 64.3 ± 7.2 | 14+6 | 3/19 | 27.6 ± 2.3 |

Lumbar punctures were performed with the patients in the sitting position according to the protocol described elsewhere (Lewczuk et al., 2006b). Briefly, after collection of the CSF for routine diagnosis (2-5 mL), additionally 4.5 mL of the CSF for this study was sampled into the same polypropylene test tube. The tube was gently shaken, and the CSF was centrifuged immediately after collection (1600g, room temperature, 15 min.) and then aliquoted into 16 polypropylene test tubes (250 µL each), and frozen within 30-40 min. after the puncture. The CSF was at no time thawed/refrozen. The analysis was performed in one center (University of Erlangen) to which all the aliquots of frozen CSF were shipped on dry ice.

### 2.2. Assays

In all the subjects of this study, routine neurochemical dementia diagnostics was performed in the CSF, including: Aβ1-42, Aβx-42, Aβx-40, Aβ Ratio, total Tau, and pTau181, according to the protocols described elsewhere (Lewczuk et al., 2004a; Lewczuk et al., 2004c). Briefly, with the assay of the Genetics Co. (Zürich, Switzerland) Aβx-42 peptide was measured, as the capturing antibody of this assay is not N-terminus-specific, and with the assay of Innogenetics (Ghent, Belgium), Aβ1-42 peptide was measured, as the capturing antibody of this assay shows N-terminus specificity. In both assays, the detecting antibodies are specific for the position 42.

sAPPα and sAPPβ CSF concentrations were measured with a multiplexing assay of Meso Scale Discovery (Gaithersburg, USA). Briefly, 150µL of the blocking buffer was added to all wells of the plate, and after one hour of incubation the wells were washed with the washing buffer. Next, 25µL of standards or samples were applied, and the plate was incubated for one hour at room temperature on a shaker. After the next washing step, the incubation followed with 25µL of the detection antibody per well at the final concentration of 1 nM. After the next washing, the reading buffer was added to all wells, and followed 10 min. of incubation the plate was read with the Sector Imager (Meso Sale Discovery). All measurements were performed in duplicate.

To test possible cross reactivity, a standard of sAPPα at the concentration of 200ng/mL was applied into two wells of the plate, and a standard of sAPPβ at the concentration of 200ng/mL was applied into two different wells of the plate. Moreover, Aβ42 synthetic peptide at the concentration of 4ng/mL, and Aβ40 synthetic peptide at the concentration of 4ng/mL were tested for cross-reactivity of sAPPα and sAPPβ assays.

To analyze intraassay imprecision, n=8 CSF samples were tested ten times on one plate, and to analyze interassay imprecision, one CSF sample was tested on five different plates during five different experiments.

Storage stability of sAPPα and sAPPβ CSF concentrations was tested with n=5 CSF samples divided after lumbar puncture into four aliquots (A-D): A, frozen and stored at -80°C for three days; B, stored at room temperature (ca. +20°C) for three days; C, stored at +4°C for three days; and D, frozen at -80°C and exposed to three freeze/thaw cycles during three days of storage. Following three days of samples' storage, sAPPα and sAPPβ in all aliquots of all five samples were measured on one plate.

### 2.3. Statistical analysis

If not stated otherwise, the results are presented as medians and interquartile ranges. Statistical differences between groups were analyzed with ANOVA followed by Scheffe post hoc test. Correlations between measured values were analyzed with Pearson's correlation factor. Differences were considered significant if p<0.05. Analyses were performed with Statistica 7.0 (Statsoft, Tulsa, USA).

### 3. Results

### 3.1. Analytical performance of the assay

Intraassay imprecision, expressed as coefficients of variation for three (out of eight) samples representing the highest, middle, and the lowest sAPPα and sAPPβ concentrations is presented in the Table 2. In the remaining five samples, imprecision turned out to be below 5% for both biomarkers (with one exception: 7.1 % in case of sAPPβ in one CSF sample).

**Table 2: Intraassay imprecision (concentrations in ng/mL)**

| Sample | sAPPα | | sAPPβ | |
|---|---|---|---|---|
| | Concentration | CV (%) | Concentration | CV (%) |
| 1. | 163.0 | 2.8 | 427.0 | 2.3 |
| 2. | 121.4 | 4.0 | 281.7 | 4.1 |
| 3. | 12.2 | 3.5 | 23.3 | 4.6 |

Interassay imprecision was 7% for sAPPα, and 9% for sAPPβ. We did not observe significant cross-reactivity between sAPPα and sAPPβ assays, and similarly we did not observe cross-reactivity of any of the two assays with either Aβ42 or Aβ40 (data not shown).

### 3.2. Stability of sAPP concentrations under different storage conditions

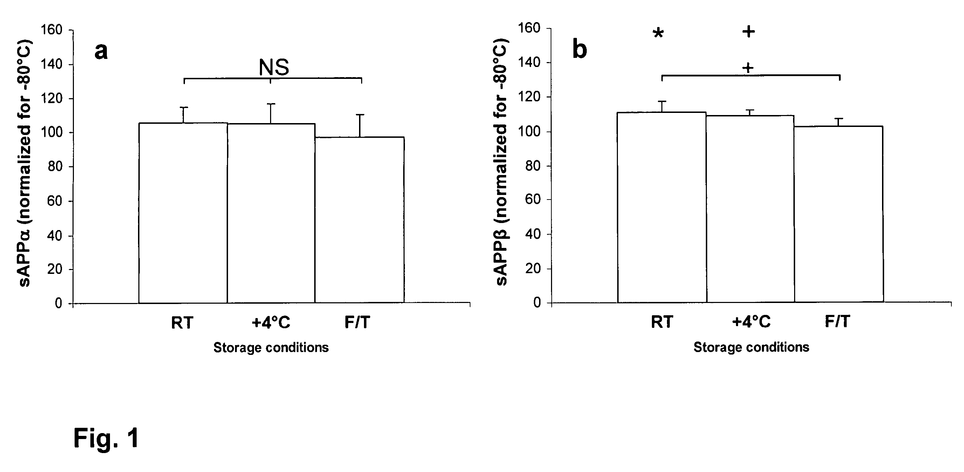

The results of storage experiments are presented in the Fig. 1 (normalized for the results of the samples stored at -80°C (aliquot A) representing 100%). Statistical analysis of the results (ANOVA for repeated measurements with *post hoc* Scheffe test) revealed no significant differences between the concentrations in the samples stored under these four conditions regarding sAPPα, but the concentrations of sAPPβ in the samples stored at -80°C turned out to be slightly lower than those stored at room temperature (p<0.01) and at +4°C (p<0.05), and the concentrations in the samples stored at room temperature were slightly higher that those of the samples exposed for repeated freeze/thaw cycles (p<0.05). Three repetitions of refreezing did not influence the results of samples stored at -80°C.

### 3.3. Correlation between sAPPα and sAPPβ CSF concentrations

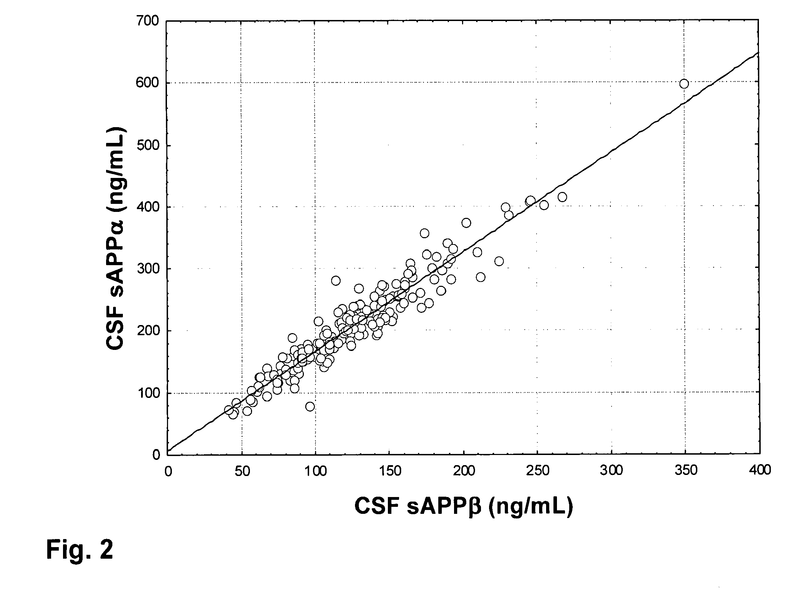

We observed very high correlation (R=0.96, p<0.001) between the CSF concentrations of both sAPP forms, as presented in the Fig. 2. sAPPα and sAPPβ weakly correlated with Aβx-40 (R=0.46 and R=0.48, respectively, p<0.001 in both cases). Correlation with other biomarkers, if at all, was only marginal (R<0.35, data not shown).

### 3.4. sAPPα and sAPPβ as potential biomarkers of Alzheimer's disease

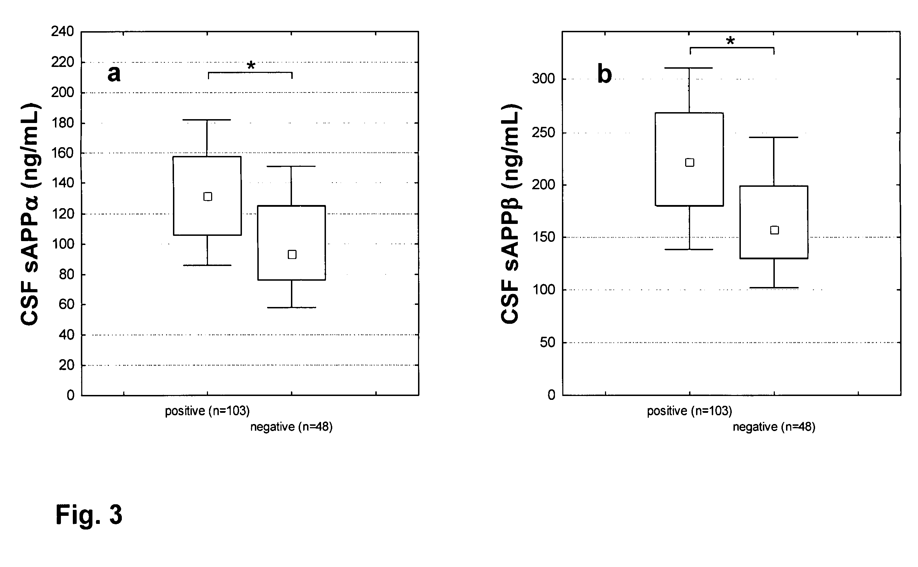

The results of the sAPPα and sAPPβ CSF concentrations in NDD+ and NDD- patients are presented in the figure 3. The concentration of both biomarkers turned out to be highly significantly (p<0.001) higher in NDD+ compared to NDD-. Interestingly, we did not observe any difference between NDD+ subjects carrying APOE ε4 allele versus those without this allele, and similarly there were no difference between NDD- subjects with and without APOE ε4 allele (data not shown).

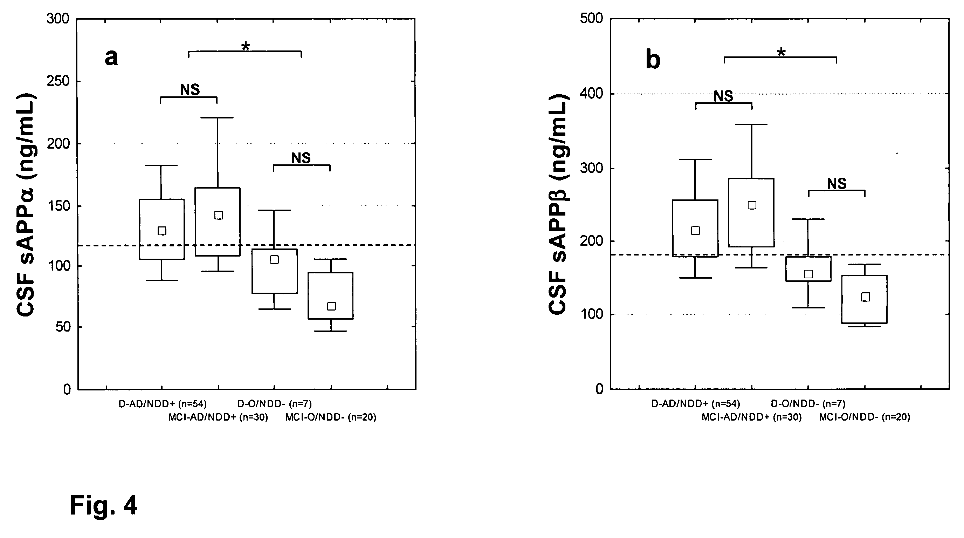

The results of sAPPα and sAPPβ CSF concentrations in patients whose clinical diagnoses were supported by neurochemical findings (i.e. Aβ Ratio and pTau181 in agreement with the clinical diagnoses) are presented in the figure 4. We observed significant differences of the concentrations of both biomarkers between D-AD/NDD+ and D-O/NDD-, as well as MCI-AD/NDD+ and MCI-O/NDD-. There were no statistical differences concerning sAPPα or sAPPβ between D-AD/NDD+ and MCI-AD/NDD+, and between D-O/NDD- and MCI-O/NDD-, respectively. We did not see statistically significant correlation between either sAPPα or sAPPβ and age of patients (data not shown). Overall, the female patients had higher sAPPα and sAPPβ concentrations compared to the male patients (data not shown).

### 3.5. Cut off values, sensitivities, and specificities

Cut off values, sensitivities, and specificities of the biomarkers (sAPPα and sAPPβ) are presented in the Table 3. For the calculation of cut offs, D-AD and MCI-AD groups were combined and the cut off values were calculated optimally separating this group from D-O and MCI-O according to the Youden's index.

**Table 3: Cut off values, sensitivities and specificities of the biomarkers**

| D-AD/NDD+ combined with MCI-AD/NDD+ versus | | | |
|---|---|---|---|
| D-O/NDD- combined with MCI-O/NDD- | | | |
| Biomarker | Cut off | Sensitivity | Specificity |
| | | [%] | [%] |
| sAPPα | 117.4ng/mL | 68 | 85 |
| sAPPβ | 181.8ng/mL | 75 | 85 |

### 4. Discussion

The presented results of a multicenter study on soluble forms of amyloid precursor proteins α and β in CSF of patients with different types of early dementia and mild cognitive impairment allow for the following conclusions.

One of the important issues in clinical neurochemistry is management of samples (human cerebrospinal fluid) that is very precious material obtained with procedure regarded as invasive. The volume of a sample is sometimes, due to e.g. technical problems, extremely small (in critical cases less than 1 mL) and the number of biomarkers to study or to measure routinely grows rapidly. To solve this conflict, different multiplexing platforms have been developed and established in laboratories enabling simultaneous measurements of more that one biomarker in one sample volume, during one analytical run, saving precious material, time, and effort (Lewczuk et al., 2007).

When establishing a new assay in a laboratory, a critical question must be addressed of reliability of the results, and as a matter of fact assays with high imprecision cannot be considered for routine use even if they promise help in diagnosis of patients. Therefore, before starting analysis of patients' samples, a set of experiments was performed to control the assay's performance, which turned out to be well acceptable (intraassay imprecision less than 5% in low-, middle-, and high concentration range, and interassay imprecision less than 10%).

Thinking in terms of routine application of the potential biomarkers, it is important to address a practical question of sample delivery and storage (Bibl et al., 2004; Lewczuk et al., 2004b; Lewczuk et al., 2006a). In case of both candidate biomarkers in this study, no difference was observed between the results from samples stored at +4°C and room temperature, and between samples constantly kept frozen (at -80°C) or exposed for re-freezing, respectively. This could mean that CSF samples may be sent between laboratories via regular mail without necessity of cooling, and that precious aliquots of experimental study samples can be thawed and refrozen. However, at least for sAPPβ, cut offs and ranges must not be mixed for frozen and non-frozen samples. The limitation of this conclusion is, however, a small number of samples studied.

The most intriguing outcomes of the presented study is the surprisingly high correlation between the CSF concentrations of the two forms of soluble APP. In such a case, an artifact has to be considered, and the explanation could be for example cross reactivity between antibodies for sAPPα and sAPPβ, and/or cross reactivity of the assay with some other CSF molecules. However, since such unspecificity of the antibodies (no cross reactivity between α and β forms) were excluded, it may be concluded that the above observation corresponds to a real physiological phenomenon. Thus, it is justified to postulate presence of some kind of hypothetical factor regulating metabolism of APP, for example by controlling activities of α and β secretases.

Highly significantly increased sAPPα and sAPPβ CSF concentration in patients with Alzheimer's disease compared to patients with other dementias were observed, if clinical diagnosis was supported by findings of routine neurochemical dementia diagnosis (amyloid β peptides, tau, pTau181). This raises a question of reliability of differential diagnosis in early dementia. It is known that clinical diagnosis in later stages of dementias might be incorrect in a considerable percentage of patients, and the diagnostic uncertainty in early cases, as these included into our study (MMSE≥20), is probably even larger. Therefore, it is justified to postulate that CSF biomarkers should be routinely measured to increase the accuracy of differential diagnosis. In a recently published report, Engelborghs et al. tested diagnostic performance of the CSF biomarkers: Aβ1-42, total tau, and pTau181 on the ground of autopsy-controlled cases, and concluded that dementia could be discriminated from controls with sensitivity of 86% and specificity of 89%, obviously showing the value of biomarkers in differential dementia diagnosis (Engelborghs et al., 2007).

The results presented here show no differences between sAPPα or sAPPβ concentrations of patients in MCI stage and those already having early dementias. This fully supports observations of the group of Blennow (Hansson et al., 2006; Zetterberg et al., 2003), as well as our recently published data (Lewczuk et al., 2007) showing alterations of CSF Aβ1-42, total tau, and pTau181 in cases of MCI subjects showing increased risk to develop AD compared with those without such risk. Considering all these reports, strong evidence indicates that the AD-indicative alterations of the CSF biomarkers can be observed years prior to the onset of dementia. This hypothesis may have high impact on prophylactics and preventive treatment of Alzheimer's disease in the future.

Summarizing, sAPPα and sAPPβ represent novel promising markers of Alzheimer's disease especially if combined with markers already routinely applied.

Many variations and modifications may be made to the preferred embodiments of the invention without departing substantially from the spirit and principles of the invention. All such modifications and variations are intended to be included herein within the scope of the present invention, as defined by the following claims.

### REFERRENCES

Andreasen N., Minthon L., Davidsson P., Vanmechelen E., Vanderstichele H., Winblad B. & Blennow K. (2001) Evaluation of CSF-tau and CSF-Ab42 as diagnostic markers for Alzheimer disease in clinical practice. Arch Neurol 58, 373-9.

Bibl M., Esselmann H., Otto M., Lewczuk P., Cepek L., Rüther E., Kornhuber J. & Wiltfang J. (2004) Cerebrospinal fluid amyloid b peptide patterns in Alzheimer's disease patients and nondemented controls depend on sample pretreatment: Indication of carrier-mediated epitope masking of amyloid beta peptides. Electrophoresis 25, 2912-8.

Blennow K., Wallin A. & Hager O. (1993) Low frequency of post-lumbar puncture headache in demented patients. Acta Neurol Scand 88, 221-3.

Engelborghs S., De Vreese K., Van de Casteele T., Vanderstichele H., Van Everbroeck B., Cras P., Martin J.J., Vanmechelen E. & De Deyn P.P. (2007) Diagnostic performance of a CSF-biomarker panel in autopsy-confirmed dementia. Neurobiol Aging.

Folstein M.F., Folstein S.E. & McHugh P.R. (1975) "Mini-mental state". A practical method for grading the cognitive state of patients for the clinician. J Psychiatr Res 12, 189-98.

Hansson O., Zetterberg H., Buchhave P., Londos E., Blennow K. & Minthon L. (2006) Association between CSF biomarkers and incipient Alzheimer's disease in patients with mild cognitive impairment: a follow-up study. Lancet Neurol 5, 228-34.

Harting C., Markowitsch H.J., Neufeld H., Calabrese P. & Deisinger K. (2000) Deutsche Adaptation der revidierten Fassung der Wechsler-Memory Scale (WMS-R) (Verlag Hans Huber, Bern, Göttingen).

Hindmarch I., Lehfeld H., de Jongh P. & Erzigkeit H. (1998) The Bayer Activities of Daily Living Scale (B-ADL). Dement Geriatr Cogn Disord 9 Suppl 2, 20-6.

Jorm A.F. (1994) A short form of the Informant Questionnaire on Cognitive Decline in the Elderly (IQCODE): development and cross-validation. Psychol Med 24, 145-53.

Klafki H.W., Staufenbiel M., Kornhuber J. & Wiltfang J. (2006) Therapeutic approaches to Alzheimer's disease. Brain 129, 2840-55.

Lewczuk P., Esselmann H., Bibl M., Beck G., Maler J.M., Otto M., Kornhuber J. & Wiltfang J. (2004a) Tau Protein Phosphorylated at Threonine 181 in CSF as a Neurochemical Biomarker in Alzheimer's Disease: Original Data and Review of the Literature. J Mol Neurosci 23, 115-22.

Lewczuk P., Esselmann H., Bibl M., Paul S., Svitek J., Miertschischk J., Meyrer R., Smirnov A., Maler J.M., Klein C., Otto M., Bleich S., Sperling W., Kornhuber J., Ruther E. & Wiltfang J. (2004b) Electrophoretic separation of amyloid beta peptides in plasma. Electrophoresis 25, 3336-3343.

Lewczuk P., Esselmann H., Otto M., Maler J.M., Henkel A.W., Henkel M.K., Eikenberg O., Antz C., Krause W.R., Reulbach U., Kornhuber J. & Wiltfang J. (2004c) Neurochemical diagnosis of Alzheimer's dementia by CSF Ab42, Ab42/Ab40 ratio and total tau. Neurobiol Aging 25, 273-81.

Lewczuk P., Beck G., Ganslandt O., Esselmann H., Deisenhammer F., Regeniter A., Petereit H.F., Tumani H., Gerritzen A., Oschmann P., Schroder J., Schonknecht P., Zimmermann K., Hampel H., Burger K., Otto M., Haustein S., Herzog K., Dannenberg R., Wurster U., Bibl M., Maler J.M., Reubach U., Kornhuber J. & Wiltfang J. (2006a) International quality control survey of neurochemical dementia diagnostics. Neurosci Lett.

Lewczuk P., Kornhuber J. & Wiltfang J. (2006b) The German Competence Net Dementias: Standard operating procedures for the neurochemical dementia diagnostics. J Neural Transm 113, 1075-80.

Lewczuk P., Kornhuber J., Vanderstichele H., Vanmechelen E., Esselmann H., Bibl M., Wolf S., Otto M., Reulbach U., Kolsch H., Jessen F., Schroder J., Schonknecht P., Hampel H., Peters O., Weimer E., Perneczky R., Jahn H., Luckhaus C., Lamla U., Supprian T., Maler J.M. & Wiltfang J. (2007) Multiplexed quantification of dementia biomarkers in the CSF of patients with early dementias and MCI: A multicenter study. Neurobiol Aging.

Mayeux R. (1998) Evaluation and use of diagnostic tests in Alzheimer's disease. Neurobiol Aging 19, 139-43.

McKhann G., Drachman D., Folstein M., Katzman R., Price D. & Stadlan E.M. (1984) Clinical diagnosis of Alzheimer's disease: report of the NINCDS-ADRDA Work Group under the auspices of Department of Health and Human Services Task Force on Alzheimer's Disease. Neurology 34, 939-44.

Morris J.C. (1993) The Clinical Dementia Rating (CDR): current version and scoring rules. Neurology 43, 2412-4.

Olsson A., Vanderstichele H., Andreasen N., De Meyer G., Wallin A., Holmberg B., Rosengren L., Vanmechelen E. & Blennow K. (2005) Simultaneous measurement of beta-amyloid(1-42), total tau, and phosphorylated tau (Thr181) in cerebrospinal fluid by the xMAP technology. Clin Chem 51, 336-45.

Petersen R.C., Doody R., Kurz A., Mohs R.C., Morris J.C., Rabins P.V., Ritchie K., Rossor M., Thal L. & Winblad B. (2001) Current concepts in mild cognitive impairment. Arch Neurol 58, 1985-92.

Reitan R.M. (1979) Trail Making Test (TMT) (Hogrefe Verlag, Bern, Göttingen).

Shulman K.I., Shedletsky R. & Silver I.L. (1986) Challenge of time: Clock-drawing and cognitive function in the elderly. Int J Ger Psychiatr 1, 135-140.

Thalmann B., Monsch A.U., Bernasconi F., Berres M., Schneitter M., Ermini-Fuenfschilling D., Spiegel R. & Staehelin H.B. (1998) Die CERAD Neuropsychologische Testbatterie - Ein gemeinsames minimales Instrumentarium zur Demenzabklärung. (Memory Clinic, Geriatrische Universitätsklinik Basel, Basel).

The Working Group on: "Molecular and Biochemical Markers of Alzheimer's Disease" (1998) Consensus report of the Working Group on: "Molecular and Biochemical Markers of Alzheimer's Disease". The Ronald and Nancy Reagan Research Institute of the Alzheimer's Association and the National Institute on Aging Working Group. Neurobiol Aging 19, 109-16.

Tynan S.H., Keller P., Lewis J., Umek R.M. & Wohlstadter J. N. (2005) Multiplex Detection of Alzheimer's Disease-related Proteins and Peptides" E-pub. on www.mesoscale.com.

Wiltfang J., Lewczuk P., Riederer P., Grünblatt E., Hock C., Scheltens P., Hampel H., Vanderstichele H., Iqbal K., Galasko D., Lannfelt L., Otto M., Esselmann H., Henkel A.W., Kornhuber J. & Blennow K. (2005) Consensus Paper of the WFSBP Task Force on Biological Markers of Dementia: The role of CSF and blood analysis in the early and differential diagnosis of dementia. World J Biol Psychiatry 6, 69-84.

Zetterberg H., Wahlund L.O. & Blennow K. (2003) Cerebrospinal fluid markers for prediction of Alzheimer's disease. Neurosci Lett 352, 67-9.

## Claims

1. An *ex vivo* method of diagnosing Alzheimer's disease comprising the steps of:
- analysing a cerebrospinal fluid (CSF) sample taken from a patient with regard to a concentration of at least one amyloid precursor protein (APP) product;
- comparing the concentration of the at least one APP product with a threshold-value for the concentration of the respective one APP product; and
- indicating a probability that the patient has or is prone to Alzheimer's disease, if the concentration of the at least one APP product exceeds the threshold-value for the concentration of the respective one APP product;
**characterized in that** the at least one APP product is selected from the group consisting of soluble forms of amyloid precursor protein α and β (sAPPα and sAPPβ).

2. The method of claim 1, **characterized in that** both the concentration of sAPPα and sAPPβ are determined and compared with a threshold-value for the concentration of the respective one APP product, and that a probability that the patient has or is prone to Alzheimer's disease is only indicated, if the concentration of both sAPPα and sAPPβ exceed the threshold-value for the concentration of the respective one APP product.

3. The method of claim 1 or 2, **characterized in that** the threshold value for the concentration of sAPPα is between 110 ng/mL and 125 ng/mL, preferably between 115 ng/mL and 120 ng/mL and more preferably at about 117.4 mg/mL.

4. The method of any of the claims 1 to 3, **characterized in that that** the threshold value for the concentration of sAPPβ is between 175 ng/mL and 190 ng/mL, preferably between 180 ng/mL and 185 ng/mL and more preferably at about 181.8 mg/mL.

5. The method of any of the claims 1 to 4, **characterized in that** the threshold value for the concentration of sAPPα displays a sensitivity of at least 60 %, preferably of at least 65 % and more preferably of at least 68 %, and a specificity of at least 75 %, preferably of at least 80 % and more preferably of at least 85 %.

6. The method of any of the claims 1 to 5, **characterized in that** the threshold value for the concentration of sAPPβ displays a sensitivity of at least 65 %, preferably of at least 70 % and more preferably of at least 75 %, and a specificity of at least 75 %, preferably of at least 80 % and more preferably of at least 85 %.

7. A method of using an immunoassay using an electrochemiluminescent reporter for determining a concentration of at least one amyloid precursor protein (APP) product selected from the group consisting of soluble forms of amyloid precursor protein α and β (sAPPα and sAPPβ) in a cerebrospinal fluid (CSF) sample in executing the method of any of the claims 1 to 5.

8. A kit for carrying out the method of any of the claims 1 to 5, comprising an assay for determining a concentration of at least one amyloid precursor protein (APP) product selected from the group consisting of soluble forms of amyloid precursor protein α and β (sAPPα and sAPPβ) in a cerebrospinal fluid (CSF) sample, **characterized in that** the kit further comprises an indicator indicating whether the concentration of the at least one APP product exceeds a threshold-value for the concentration of the respective one APP product.

9. The kit of claim 8, **characterized in that** the assay comprises the necessary components for determining the concentrations of both sAPPα and sAPPβ, and that the indicators indicates whether the concentrations of both sAPPα and sAPPβ exceed their respective threshold-value.

10. The kit of claim 8 or 9, **characterized in that** the threshold value for the concentration of sAPPα is between 110 ng/mL and 125 ng/mL, preferably between 115 ng/mL and 120 ng/mL and more preferably at about 117.4 mg/mL, and that the threshold value for the concentration of sAPPβ is between 175 ng/mL and 190 ng/mL, preferably between 180 ng/mL and 185 ng/mL and more preferably at about 181.8 mg/mL.
